# FASCICULE DE BREVET EUROPEEN

(11) **EP 1 455 693 B1**
(45) Date de publication et mention de la délivrance du brevet: **04.04.2007**
(21) Numéro de dépôt: 02791891.1
(22) Date de dépôt: 24.10.2002
(51) Int. Cl.: A61F 2/36, A61F 2/30, A61F 2/46

(54) **GAMME DE TIGES FEMORALES FIXEES PAR UN LIQUIDE INJECTE PAR LEUR CENTRE**
SET AUS FEMURSCHÄFTEN, DIE MIT EINER DURCH IHRE MITTE INJIZIERTEN FLÜSSIGKEIT FIXIERT WERDEN
SET OF FEMORAL STEMS FIXED WITH A LIQUID INJECTED THROUGH THEIR CENTER

(30) Priorité: 24.10.2001 FR 0114073
(43) Date de publication de la demande: 15.09.2004
(73) Titulaire: Société de Génie Médical - SGM, 42000 Saint Etienne (FR)
(72) Inventeur: DURAND, Alain, F-42300 Roanne (FR); DUTHOIT, Etienne, F-59830 Louvil (FR); FINET, Pierre, F-38240 Meylan (FR); FRESSYNET, René, F-26100 Romans (FR); GHESTEM, Didier, F-62580 Neuville St Vaast (FR); GUERIOT, Sylvain, F-59500 Douai (FR); LEDOYEN, Christian, F-69400 Gleize (FR); LEGRAND, Jean-Jacques, F-73000 Chambery (FR); MIGNOT, Pierre, F-26500 Bourg les Valence (FR); MORILLEAU, Philippe, F-38240 Meylan (FR); QUANDALLE, François, F-62580 Vimy (FR); THOMAS, Régis, F-62000 Arras (FR)
(74) Mandataire: Thivillier, Patrick
(86) Numéro de dépôt international: PCT/FR2002/003641
(87) Numéro de publication internationale: WO 2003/034954

(56) Documents cités:
- EP-A- 0 379 785
- EP-A- 0 434 604
- EP-A- 0 995 411
- WO-A-93/08769
- DE-A- 3 314 210
- DE-A- 19 613 081
- DE-A- 19 740 755
- FR-A- 2 705 884
- FR-A- 2 720 268
- FR-A- 2 728 160
- FR-A- 2 758 977
- FR-A- 2 793 675
- GB-A- 2 344 052
- US-A- 5 340 362

## Description

L'invention se rattache au secteur technique des implants orthopédiques, notamment des tiges fémorales pour prothèses de hanches.

D'une manière parfaitement connue, une tige fémorale est destinée à être impactée dans le canal médullaire du fémur. L'extrémité proximale de la tige reçoit, directement ou d'une manière rapportée, une tête fémorale coopérant avec la cavité cotyloïdienne de l'os iliaque. Parmi ces différentes tiges fémorales, il convient de distinguer celles conformées pour être impactées avec du ciment, et celles conformées pour être impactées sans ciment. Les tiges cimentées sont utilisées soit de façon systématique par certains praticiens, soit en alternance avec des tiges sans ciment, voire en secours, par d'autres praticiens, quand la forme ou la qualité osseuse ne se prête pas à une implantation sans ciment.

L'opérateur ne pouvant pas toujours prévoir avant l'intervention quel type d'implantation, cimentée ou sans ciment, il va devoir pratiquer, il est apparu important de pouvoir disposer, avec la même instrumentation de râpes, des 2 versions de tige susceptibles d'être impactées avec ou sans ciment, avec dans tous les cas, le meilleur mode de fixation de l'implant à l'os.

Le degré de difficulté du passage des râpes de préparation osseuse et la stabilité de la dernière râpe utilisée étant en effet indicatifs de la nécessité de cimenter ou non l'implant définitif.

Une solution avantageuse ressort de l'enseignement du brevet FR 2.793.675. Ce brevet concerne un jeu de tiges fémorales comprenant, pour une même taille, une tige agencée pour être impactée avec du ciment, et une tige pour être impactée sans ciment. La section transversale de chacune des tiges, considérée au niveau métaphysaire, est différente et déterminée pour permettre leur impaction avec une même râpe qui est identique en dimensions à la tige sans ciment. La section transversale de la tige sans ciment est définie pour assurer un remplissage métaphysaire optimal, tandis que la section transversale de la tige cimentée est définie pour assurer à la fois sa stabilité et la mise en compression localisée, au niveau d'évidements latéraux, d'une couche de ciment d'épaisseur régulière.

Selon cette solution, il n'apparaît pas de moyen mis en oeuvre pour améliorer 2 autres paramètres importants pour la longévité de la fixation d'une tige cimentée : son auto-centrage diaphysaire et l'homogénéité de composition de la couche cimentée. La tige est en effet mise en place sans guide diaphysaire calibré dans le fémur préalablement injecté de ciment selon la méthode classique de cimentage. Ce mode de cimentage laisse inclus dans le fourreau de ciment des bulles d'air et des résidus sanguins et graisseux affaiblissant à terme les caractéristiques de résistance dudit fourreau.

On peut également citer l'enseignement du brevet FR 2728160 qui décrit un système de prothèse de hanche à tiges cimentée et sans ciment utilisant une râpe universelle. Les tiges sont destinées à être indifféremment implantées dans la cavité fémorale réalisée au moyen de la râpe qui comporte un corps de section déterminée sur laquelle sont formées des dents abrasives. Ce document ne donne aucune indication permettant un centrage optimale de la tige en partie métaphysaire tout en autorisant un remplissage en ciment de la partie diaphysaire et de la partie métaphysaire.

A partir de cet état de la technique, notamment pour éviter de créer des inhomogénéités de composition et d'épaisseur de la couche de ciment et pour diminuer substantiellement le cisaillement de cette couche de ciment sous charge, il est apparu avantageux, dans un premier temps, de mettre en place la tige fémorale et, dans un deuxième temps, d'injecter le ciment et de remplir l'espace entre la tige et l'os, limité distalement par un bouchon-centreur-diffuseur et proximalement par une section de tige remplissant complètement l'espace intramédullaire.

Pour résoudre un tel problème, il a été conçu et mis au point un ensemble de tiges fémorales conformes aux caractéristiques de la revendication 1

Pour résoudre le problème posé d'assurer la rigidification du bouchon-obturateur-centreur monté à l'extrémité de l'implant au moment de leur introduction dans la cavité osseuse, le bouchon reçoit, avec capacité de démontage, une tige de rigidification destinée à coopérer avec le canal central de la tige fémorale et vissée au centre du bouchon.

Pour résoudre le problème posé de remplir l'espace entre la tige et l'os, le bouchon est engagé en bout de l'extrémité distale de la tige fémorale et présente des trous pour l'évacuation du ciment et sa remontée dans la cavité osseuse, lesdits trous étant situés entre ladite extrémité distale et des ailettes de centrage et d'obturation que présente ledit bouchon.

Pour résoudre le problème posé d'assurer l'injection en tant que telle du ciment, le canal central d'injection du ciment est prolongé en partie proximale de la tige d'un chambrage taraudé pour le vissage d'un connecteur apte à permettre l'injection du ciment en reliant l'extrémité de la seringue d'injection du ciment au trou central de l'implant.

Selon une autre caractéristique, le chambrage taraudé reçoit, par vissage, un organe obturateur du canal central prolongé par une tige apte à assurer, après écoulement du ciment, le nettoyage du canal central de la tige.

Pour résoudre le problème posé d'évacuer toute impureté hors de la couche cimentée, le bloc de centrage et de pression métaphysaire de la tige présente une limite inférieure arciforme dont le sommet de concavité est creusé d'évents d'évacuation du ciment résiduel et autres impuretés nuisant à l'homogénéité de composition de la couche de ciment (air, graisse, sang ...).

En ce qui concerne la tige fémorale destinée à être impactée sans ciment, cette dernière est conformée en section pour assurer un remplissage métaphyso-diaphysaire optimal du fémur et un auto-blocage immédiat à l'impaction.

L'invention est exposée ci-après plus en détail à l'aide des figures des dessins annexés dans lesquels :

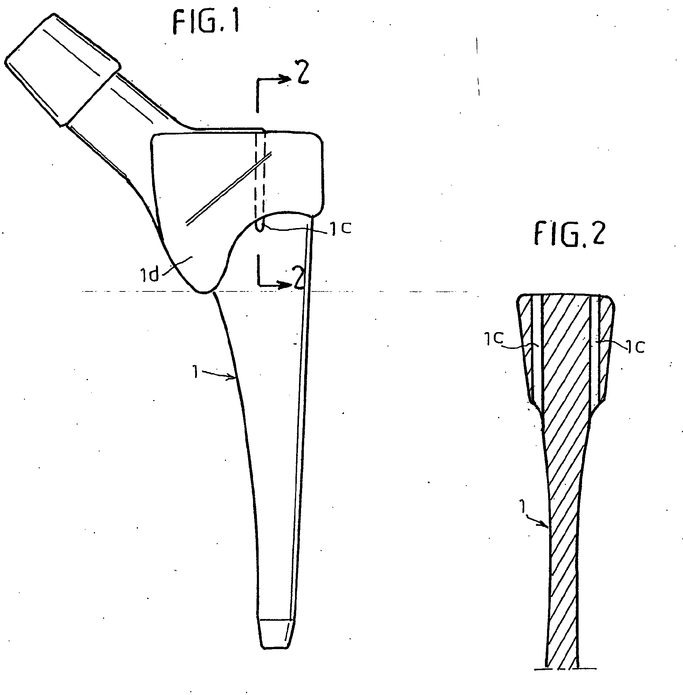

- la figure 1 est une vue de face de la tige fémorale cimentée ;
- la figure 2 est une vue en coupe longitudinale considérée selon la ligne 2-2 de la figure 1
- la figure 3 est une vue de face de la tige fémorale non cimentée ;
- la figure 4 est une vue de face de la tige fémorale cimentée et de la râpe (de même dimension que la tige non cimentée) de taille équivalente superposées montrant l'espace ménagé pour le ciment ;
- les figures 5, 6, 7, 8 montrent les principales phases opératoires pour la mise en place de la prothèse cimentée.

Selon l'invention, pour chaque taille de râpe, la gamme de tiges fémorales comprend une tige (1) agencée pour être impactée avec du ciment et une tige (2) conformée pour être impactée sans ciment.

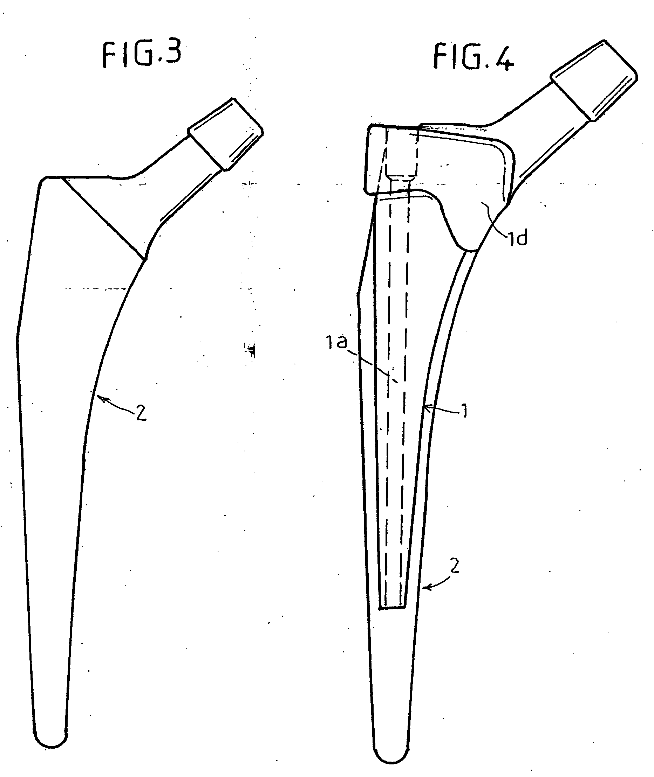

En ce qui concerne la tige (2) conformée pour être impactée sans ciment, cette dernière est du type de celle décrite et illustrée dans le brevet précité FR 2.793.675. La figure 3 montre un exemple d'une tige fémorale (2) conformée pour être impactée sans ciment. Les profils en section de cette tige sont déterminés pour assurer un remplissage métaphyso-diaphysaire optimal et un auto-blocage immédiat à l'impaction.

La tige (1) destinée, selon l'invention, à être impactée avec du ciment est autocentrée en partie métaphysaire par une portion (1d) au niveau de coupe cervicale identique en dimensions frontales et sagittales à la râpe et centrée en partie diaphysaire par un bouchon obturateur (3). Par ailleurs, la tige fémorale (1), présente un canal central débouchant (1a) coopérant avec le bouchon obturateur (3) pour l'injection du ciment et son refoulement à l'extérieur en vue de remplir l'espace entre la tige (1) et l'os.

Le bouchon (3) est avantageusement réalisé en polyéthylène. Ce bouchon présente une partie cylindrique conformée intérieurement pour être emmanchée à l'extrémité distale de la tige fémorale (1). La paroi inférieure de la partie (3a) est percée de trous disposés circulairement (3b) pour permettre l'évacuation du ciment (c) et son refoulement, comme il sera indiqué dans la suite de la description, dans la cavité fémorale. La partie cylindrique (3a) est prolongée par des ailettes (3c) conformées pour obturer parfaitement la cavité fémorale tout en assurant le centrage de la tige (1) dans le canal.

Après mise en place du bouchon (3), à l'extrémité distale de la tige fémorale (1), les trous (3b) sont situés entre ladite extrémité distale percée du canal d'écoulement du ciment et les ailettes d'obturation (3c) du bouchon (3).

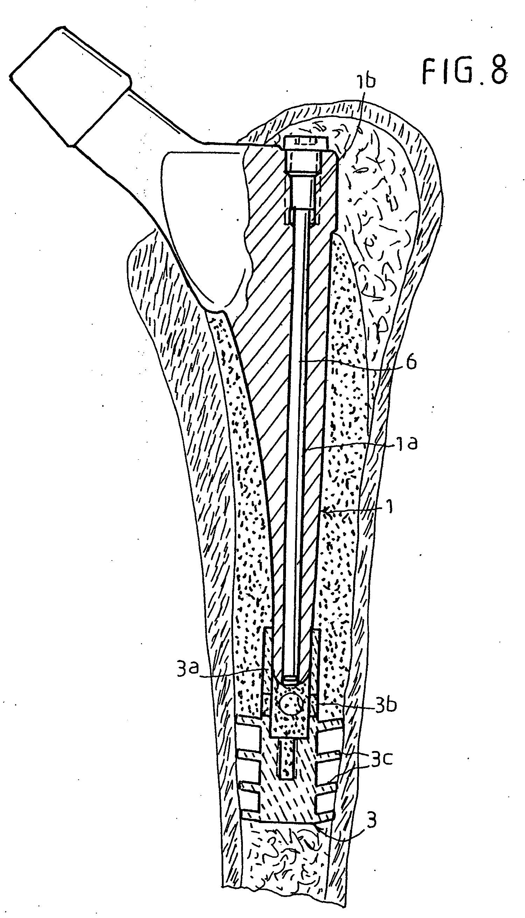

La mise en place de la tige fémorale (1) cimentée s'effectue, comme indiqué ci-après, en se référant plus particulièrement aux figures 5, 6, 7 et 8.

La tige fémorale (1) est préalablement équipée du bouchon obturateur (3) dont la taille a été déterminée par un calibrage de la diaphyse fémorale. Une tige de rigidification (4) est alors engagée dans le canal central (1a) de la tige fémorale (1) pour être vissée par son extrémité filetée (4a), dans un trou taraudé (3d) au centre du bouchon (3). Cette tige de rigidification (4) peut présenter un prolongement (4b) à l'extérieur du fût servant à guider et impacter la tige fémorale dans le fût.

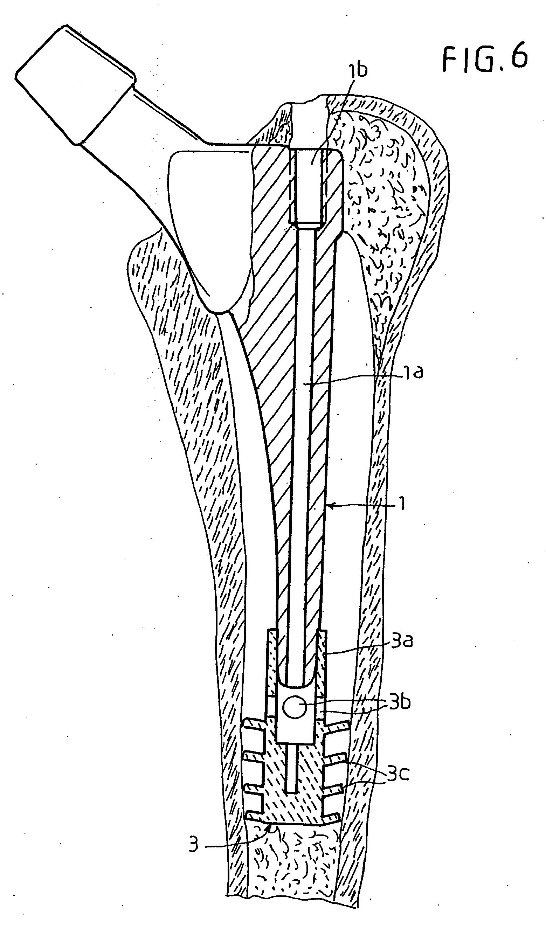

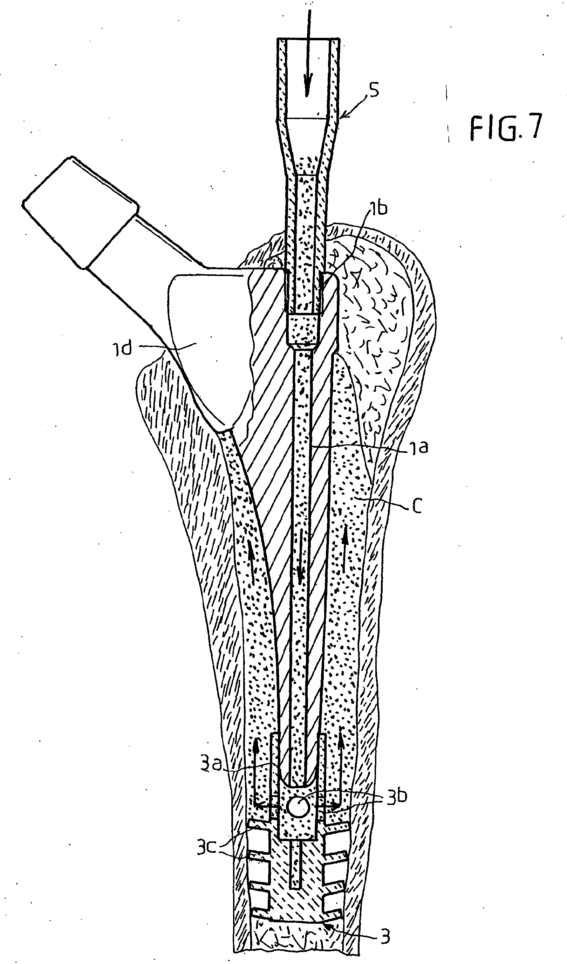

Une fois la tige fémorale en place, l'opérateur peut dévisser la tige de rigidification (4) pour procéder à l'injection du ciment (C) au-travers du canal (1a) (figure 6). Pour cela, l'opérateur utilise un connecteur (5) vissé dans un chambrage taraudé (1b) à l'extrémité proximale du canal central de l'implant. Le connecteur (5), à son autre extrémité, est relié à l'embout d'une seringue à ciment, matériel connu pour l'injection du ciment selon le procédé classique de cimentage. Le ciment (C) est donc injecté dans le canal central (1a) en étant refoulé au niveau de l'extrémité distale de la tige (1) à travers les trous (3b) du bouchon obturateur (3). Les ailettes (3c) assurant l'obturation de la cavité osseuse, le ciment aura naturellement tendance à remonter afin de remplir l'espace entre la tige (1) et l'os avant de rencontrer l'obstacle constitué par la portion (1d) de tige occupant tout l'espace intra-médullaire constituant ainsi un bloc de contre-pression (figure 7).

Le canal central (1a) peut ensuite être nettoyé et obturé par une tige (6) dont l'extrémité proximale, sous forme de bouchon, se visse dans le chambrage taraudé (1b) de la tige fémorale (1).

A noter que la portion (1d) constituant un élément de centrage de l'implant et de pression sur le fourreau de ciment présente, au sommet concave de sa limite inférieure arciforme (1e), des évents (1c) pour l'évacuation du ciment résiduel et autres impuretés (air, graisse, sang, ...).

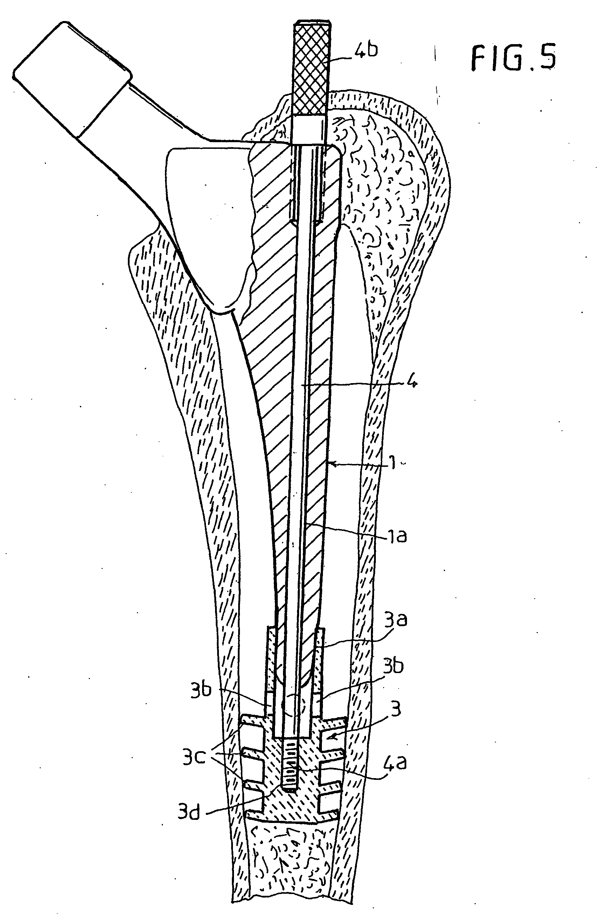

On rappelle que la section transversale de la tige cimentée (1) correspond sur une portion métaphysaire à hauteur de la résection cervicale à la section transversale, d'une râpe de même taille nominale correspondant elle-même à la section transversale de la tige sans ciment (2) de même taille (figure 4).

Sans pour cela sortir du cadre de l'invention, la tige sans ciment (2) peut présenter un canal central débouchant pour l'écoulement d'un liquide de remplissage ou de fixation. En effet, dans des cas de reprise pour descellement ou fracture osseuse, il peut être avantageux de pouvoir injecter, dans le canal central de la tige sans ciment, un ciment biologique apte à combler les pertes osseuses ou à favoriser la synthèse de l'os facturé.

Les avantages ressortent bien de la description.

## Revendications

1. Ensemble de tiges fémorales comprenant une tige (1) agencée pour être impactée dans une cavité osseuse avec du ciment, une tige (2)agencée pour être impactée dans ladite cavité sans ciment, et une râpe qui est identique en dimension à la tige sans ciment (2),
la section transversale de chacune des tiges (1) et (2) étant différente et déterminée pour permettre leur mise en place après le passage de la râpe, la section transversale de la tige sans ciment (2) étant déterminée pour assurer son auto-blocage immédiat à l'impaction, **caractérisé en ce que :**
- la tige (1) agencée pour être impactée avec du ciment est autocentrée en partie métaphysaire par une portion au niveau de la coupe cervicale identique en dimensions frontale et sagittale à la râpe et centrable en partie diaphysaire par un bouchon obturateur (3), ladite tige (1) présentant un canal central débouchant (1a) pour l'injection du ciment et son refoulement à l'extérieur pour remplir l'espace entre la tige et l'os.

2. Ensemble selon la revendication 1, **caractérisé en ce que** l'ensemble comprend un bouchon obturateur (3) et le bouchon (3) reçoit, avec capacité de démontage, une tige de rigidification (4) destinée à coopérer avec le canal central (1a) de la tige fémorale (1) et vissée au centre dudit bouchon (3).

3. Ensemble selon la revendication 1, **caractérisé en ce que** l'ensemble comprend un bouchon obturateur (3) et le bouchon (3) est engagé en bout de l'extrémité distale de la tige fémorale (1) et présente des trous (3b) pour l'évacuation du ciment ou de tout type de produit de remplissage ou de fixation osseuse et sa remontée dans la cavité osseuse, lesdits trous étant situés entre ladite extrémité distale et des ailettes (3c) de centrage et d'obturation que présente ledit bouchon.

4. Ensemble selon la revendication 1, **caractérisé en ce que** le canal central (1a) d'injection du ciment est prolongé en partie proximale de la tige (1) par un chambrage taraudé (1b) pour le vissage d'un connecteur (5) apte à permettre l'injection du ciment en reliant l'extrémité d'une seringue d'injection du ciment au canal central (1a) de l'implant.

5. Ensemble selon la revendication 4, **caractérisé en ce que**, le chambrage taraudé (1b) reçoit, par vissage, un organe obturateur (6a) du canal central prolongé par une tige (6) apte à assurer, après écoulement du ciment, le nettoyage du canal central (1a) de la tige.

6. Ensemble selon la revendication 1, **caractérisé en ce que** le bloc de centrage et de pression métaphysaire de la tige (1) présente une limite inférieure arciforme (1e) dont le sommet de concavité est creusé d'évents (1c) d'évacuation du ciment résiduel et autres impuretés nuisant à l'homogénéité de composition de la couche de ciment.

7. Ensemble selon la revendication 1, **caractérisé en ce que** la tige (2) est conformée en section pour assurer un remplissage métaphyso-diaphysaire optimal du fémur et un auto-blocage immédiat à l'impaction.

8. Ensemble selon la revendication 1, **caractérisé en ce qu'**en variante, la tige sans ciment (2) présente un canal central débouchant en vue de l'écoulement d'un liquide de remplissage ou de fixation.

## Claims

1. A set of femoral stems comprising a rod (1) designed to be impacted into a bone cavity with cement and a rod (2) designed to be impacted in said bone cavity without cement and a rasp which is identical in size to the rod without cement (2), the cross-section of each of the rods (1) and (2) being different and determined to allow their placing after passage of the rasp, the cross-section of the rod without cement (2) being determined to ensure its immediate self-locking on impacting,
**characterised in that**:
- rod (1) designed to be impacted with cement is self-centred in the metaphyseal part by a portion at the level of the cervical section identical in front and sagittal dimensions to the rasp and capable of being centred in the diaphyseal part by a sealing plug (3), said rod (1) having a central emerging cavity (1a) for injection of cement and its flow to the outside in order to fill the space between the rod and the bone.

2. A set as claimed in claim 1, **characterised in that** the set comprises a sealing plug (3) and plug (3) removably receives a rigidifying rod (4) intended to cooperate with central cavity (1a) of femoral rod (1) and screw fitted into the centre of said plug (3).

3. A set as claimed in claim 1, **characterised in that** the set comprises a sealing plug (3) and plug (3) is fitted in the tip of the distal end of femoral rod (1) and has holes (3b) for removal of cement or any type of bone filling or fixing product and its upward flow into the bone cavity, said holes being located between said distal end and the centring and sealing fins (3c) of said plug.

4. A set as claimed in claim 1, **characterised in that** central cavity (1a) for injecting the cement extends in the proximal part of rod (1) as a threaded recess (1b) for screw fastening a connector (5) capable of enabling injection of cement by linking the end of a cement injection syringe to central cavity (1a) of the implant.

5. A set as claimed in claim 4, **characterised in that** threaded recess (1b) accommodates, by screw fastening, a device for sealing (6a) the central cavity extended by a rod (6) capable of ensuring, after flow of the cement, cleaning of central cavity (1a) of the rod.

6. A set as claimed in claim 1, **characterised in that** the metaphyseal pressure and centring block of rod (1) has a lower arch-shaped limit (1e) the concave apex of which is hollowed with vents (1c) for removal of residual cement and other impurities detrimental to the homogeneity of the composition of the cement layer.

7. A set as claimed in claim 1, **characterised in that** rod (2) has a cross-section shaped to ensure optimal metaphyseal-diaphyseal filling of the femur and immediate self-locking on impacting.

8. A set as claimed in claim 1, **characterised in that** an alternative rod without cement (2) has an emerging central cavity with a view to allowing flow of a filling or fixing liquid.

## Patentansprüche

1. Set aus Femurschäften, einen Schaft (1) umfassend, der dazu angepasst ist, mit Zement in einen Knochenhohlraum impaktiert zu werden, einen Schaft (2), der dazu angepasst ist, ohne Zement in diesen Hohlraum impaktiert zu werden und eine Raspel, deren Größe identisch ist mit der des Schafts ohne Zement (2), wobei die Querschnitte der Schäfte (1) und (2) unterschiedlich sind und so bestimmt, dass sie die Platzierung der Schäfte nach dem Ausraspeln ermöglichen und wobei der Querschnitt des Schafts ohne Zement (2) so bestimmt ist, dass seine unmittelbare Selbstblockierung bei der Impaktierung sichergestellt ist, **dadurch gekennzeichnet, dass** der Schaft (1), der dazu angepasst ist, mit Zement impaktiert zu werden, im metaphysären Abschnitt selbstzentrierend ist durch einen Teil im Bereich des zervikalen Schnitts, dessen frontale und sagittale Abmessungen identisch sind mit denen der Raspel und dass dieser Schaft im diaphysären Abschnitt zentrierbar ist durch einen Verschlussstopfen (3), wobei der Schaft (1) einen zentralen ausmündenden Kanal (1a) aufweist für das Injizieren des Zements und seine Verdrängung nach außen zum Füllen des Zwischenraums zwischen dem Schaft und dem Knochen.

2. Set nach Anspruch 1, **dadurch gekennzeichnet, dass** das Set einen Verschlussstopfen (3) umfasst und der Stopfen (3) einen Schaft zur Versteifung (4) demontierbar aufnimmt, der für das Zusammenwirken mit dem zentralen Kanal (1a) des Femurschafts (1) vorgesehen ist und der in der Mitte des Stopfens (3) eingeschraubt ist.

3. Set nach Anspruch 1, **dadurch gekennzeichnet, dass** das Set einen Verschlussstopfen (3) umfasst und der Stopfen (3) mit der Spitze des distalen Endes des Femurschafts (1) verbunden ist und Löcher (3b) für den Ausfluss des Zements oder jeglicher Art von Produkt zur Knochenfüllung oder Knochenfixation in den Knochenhohlraum und das Aufsteigen in dem Knochenhohlraum aufweist, wobei die Löcher zwischen dem distalen Ende und den Zentrierungs- und Verschlussflügeln (3c), die der Stopfen aufweist, angeordnet sind.

4. Set nach Anspruch 1, **dadurch gekennzeichnet, dass** der zentrale Kanal (1a) für das Injizieren des Zements am proximalen Ende des Schafts (1) durch eine Bohrung mit Innengewinde (1b) zum Einschrauben eines Verbindungsstücks (5) verlängert ist, das geeignet ist, das Injizieren des Zements zu ermöglichen, indem es das Ende einer Spritze für die Zementinjektion mit dem zentralen Kanal (1a) des Implantats verbindet.

5. Set nach Anspruch 4, **dadurch gekennzeichnet, dass** die Bohrung mit Innengewinde (1b) durch Einschrauben ein durch einen Schaft (6) verlängertes Schließorgan (6a) [sic] des zentralen Kanals aufnimmt, wobei der Schaft dazu geeignet ist, nach dem Abfließen des Zements die Reinigung des zentralen Kanals (1a) des Femurschafts sicherzustellen.

6. Set nach Anspruch 1, **dadurch gekennzeichnet, dass** der metaphysäre Block für die Zentrierung und den Druck des Schafts (1) eine bogenförmige untere Begrenzung (1e) [sic] aufweist, wobei am Scheitelpunkt der Konkavität Abflussöffnungen (1c) für das Abfließen des Restzements und anderer, die homogene zusammensetzung der Zementschicht gefährdende Verunreinigungen ausgebohrt sind.

7. Set nach Anspruch 1, **dadurch gekennzeichnet, dass** der Schaft (2) im Querschnitt so geformt ist, dass eine optimale metaphysär-diaphysäre Ausfüllung des Femur und eine unmittelbare Selbstblockierung bei der Impaktierung sichergestellt sind.

8. Set nach Anspruch 1, **dadurch gekennzeichnet, dass** der Schaft ohne Zement (2) alternativ einen zentralen ausmündenden Kanal für das Abfließen einer Füll- oder Fixationsflüssigkeit aufweist.
